# EUROPEAN PATENT SPECIFICATION

(11) **EP 4 475 747 B1**
(45) Date of publication and mention of the grant of the patent: **02.09.2026**
(21) Application number: 23702840.2
(22) Date of filing: 06.02.2023
(51) Int. Cl.: A61B 5/021, A61B 5/352

(54) **METHOD AND SYSTEM FOR MEASURING PULSE WAVE VELOCITY**
VERFAHREN UND SYSTEM ZUR MESSUNG DER PULSWELLENGESCHWINDIGKEIT
PROCÉDÉ ET SYSTÈME DE MESURE DE LA VITESSE DES ONDES D'IMPULSION

(30) Priority: 07.02.2022 EP 22305129
(43) Date of publication of application: 18.12.2024
(73) Proprietor: Assistance Publique - Hôpitaux de Paris, 75012 Paris (FR)
(72) Inventor: VALLÉE, Fabrice, 75010 PARIS (FR); LOPES, Jean-Paul, 75010 PARIS (FR); MILLASSEAU, Sandrine, 75010 PARIS (FR); HONG, Alex, 75010 PARIS (FR); JOACHIM, Jona, 75010 PARIS (FR); MATEO, Joaquim, 75010 PARIS (FR)
(74) Representative: Flesselles, Bruno F.G.
(86) International application number: PCT/EP2023/052878
(87) International publication number: WO 2023/148386

(56) References cited:
- THE REFERENCE VALUES FOR ARTERIAL STIFFNESS' COLLABORATION: "Determinants of pulse wave velocity in healthy people and in the presence of cardiovascular risk factors: 'establishing normal and reference values'", vol. 31, no. 19, 1 October 2010 (2010-10-01), GB, pages 2338 - 2350, XP055943455, ISSN: 0195-668X, Retrieved from the Internet <URL:https://academic.oup.com/eurheartj/article-lookup/doi/10.1093/eurheartj/ehq165> DOI: 10.1093/eurheartj/ehq165
- BEUTEL FABIAN ET AL: "Pulse Arrival Time Segmentation Into Cardiac and Vascular Intervals - Implications for Pulse Wave Velocity and Blood Pressure Estimation", IEEE TRANSACTIONS ON BIOMEDICAL ENGINEERING, IEEE, USA, vol. 68, no. 9, 29 January 2021 (2021-01-29), pages 2810 - 2820, XP011873363, ISSN: 0018-9294, [retrieved on 20210818], DOI: 10.1109/TBME.2021.3055154
- JANNE M. J. HUTTUNEN ET AL: "Pulse transit time estimation of aortic pulse wave velocity and blood pressure using machine learning and simulated training data", PLOS COMPUTATIONAL BIOLOGY, vol. 15, no. 8, 15 August 2019 (2019-08-15), pages e1007259, XP055699254, DOI: 10.1371/journal.pcbi.1007259

## Description

The invention pertains to a method and system for measuring pulse wave velocity (PWV), using the pulse arrival time (PAT) and the carotid-femoral distance, but also taking the age of the patient into consideration.

### PRIOR ART

Cardiovascular mortality is one of the leading causes of death worldwide. Assessment of the stiffness of large arteries is an early and independent predictor of cardiovascular events. Indeed, arterial aging is marked by a decrease in the elasticity characteristic of large-caliber arteries.

The best parameter for the evaluation of this arterial stiffness is the measurement of the carotid-femoral pulse wave velocity (herein designated as PWVcf or PWV). The PWVcf corresponds to the propagation speed of the pulse wave from the carotid artery to the femoral artery, *i.e.* the time it takes for the pulse wave to travel from a point in the carotid artery to a point in the femoral artery.

Thus, in a patient with flexible arteries, *i.e.* a young patient with low cardiovascular risk, the PWVcf will be low (6 m/sec), whereas in a patient with more rigid arteries, *i.e.* an elderly patient at higher cardiovascular risk, it will be higher (15 m/sec).

Thus, the assessment of arterial stiffness by PWcf has become a high-priority therapeutic target, as demonstrated by its inclusion in the European recommendations for the management of hypertension. However, despite unanimous recognition by learned societies of the importance of this measurement, PWVcf has not become widespread in routine clinical practice.

One can find at least two reasons for this low adoption by the physicians:
The first one is that the measurement is relatively difficult to perform. Indeed, in clinical practice, it is essentially performed today by imaging (MRI), by ultrasound technique (combined carotid and femoral Doppler) or finally by combined tonometry (e.g. SphygmoCor, Complior). This implies specific equipment associated with training, expertise and learning of the techniques for collecting and exploiting the data. Concerning the measurement of PWV by MRI, one of the three methods is generally performed (transit-time analysis, flow-area and cross-correlation). All three methods must be performed in a radiology center with an MRI capable of performing complex imaging sequences (phase contrast MRI), are time-consuming (45 to 60 minutes) and expensive. For all these reasons, MRI can't be used a technique of choice for mass screening and testing of individuals.

The ultrasound and tonometry techniques are both based on the following formula: PWVcf = carotid-femoral distance / carotid-femoral transit time. The carotid-femoral distance is first measured with a tape measure. The carotid-femoral transit time is then measured by simultaneously recording the signals of the carotid and femoral arteries, over several beats. These carotid and femoral signals can be measured by various techniques: Doppler, tonometry, oscillometry or a combination of both (using a tonometer placed in the carotid artery and a tension cuff placed on the upper thigh to capture the femoral blood pressure signal, the two signals also being synchronized over several beats) Thus, to obtain a PWVcf measurement with these methods, an ultrasound scanner or a specific device (Sphygmocor XCEL) and a person experienced in the technique are required. Therefore, just like MRI techniques, for the same reasons but to a lesser extent, those based on ultrasound or tonometry are not suitable for large-scale screening of high-risk populations.

The second reason is the relative difficulty of interpreting the values obtained.

Indeed, the PWV values collected should be analyzed by taking into account the anthropometric data of the patient, including his age, but also the value of the blood pressure at the time of the examination. Indeed, the Bramwell-Hill equation shows a direct link between PWV and arterial distensibility (defined by the variations in the diameter of the arterial lumen) as a function of its pressure variations according to the following formula: PWV= √ [(A/ρ) (∂A/∂P)] with A=arterial lumen area ρ=blood density and ∂A/∂P=change in arterial lumen area with changes in pressures. Thus, when the arterial pressure increases, the diameter of the arterial lumen also increases according to the stiffness-distensibility (based on the elastin/collagen ratio) of the vessel. This also leads to an increase in PWV.

One can thus understand why a single PWVcf measurement at a given time cannot by itself characterize the global arterial stiffness of a patient, especially if the arterial pressure is not known. Thus, to accurately explore the arterial stiffness of a patient, it would be ideal to have PWVcf values over a wide range of blood pressure levels, which is not readily feasible with the methods currently available.

In view of the above, and in addition to the fact that measurement of PWVcf is already complicated, obtaining a large variation in blood pressure in a clinical routine to assess the variations would make the task even more difficult and ethically inadvisable because it would mean administering drugs that would cause hypo or hypertension in the patient. Some authors have tried to come closer to this goal by measuring PWVcf in the standing and lying position, with very relative success because the variations in blood pressure obtained were not very important.

In summary, for at least these two reasons, PWV is only rarely measured in daily practice.

Therefore, the development of a procedure that would allow a continuous measurement of PWVcf (multiple measures at multiple time points), and more preferably that would also link it to blood pressure variations, could be a major advance for cardiovascular risk stratification because it would allow a dynamic, personalized, and therefore more accurate analysis of arterial stiffness.

The PWV can be estimated by measuring arrivals of pulse waves at two arterial sites: $PWV = \frac{distance between the sites}{travel time between the sites}$

The travel time is commonly referred as pulse transit time (PTT).

Various authors have proposed to approximate the pulse arrival time (PAT) as the PTT and to use it, in particular on signals obtained by photo-plethysmography (PPG) to calculate the PWV. State-of-the-art estimation of PAT consists of detecting a characteristic point within the PPG waveform.

However, PAT = PEP + PTT where PEP is the pulse ejection period, which is the time elapsed between the electrical depolarization of the left ventricle (QRS on the electrocardiogram (ECG)) and the beginning of ventricular ejection and represents the period of left ventricular contraction with the cardiac valves closed.

In view of the variations in pre-ejection period (PEP), approximating the PTT by using the PAT may lead to unreliable results for PWV, and the formula that would only use the PAT and the distance (generally the carotid-femoral distance, Dcf) may thus not be accurate.

Huttunen et al (PLoS Comput Biol. 2019 Aug; 15(8): e1007259) have proposed to use machine learning and simulated training data to calculate PWV.

Solà et al (Physiol. Meas. 30 (2009) 603-615) have proposed to define new family of PAT estimators based on the whole pressure pulse waveform through a specific parametric model, considering that using characteristic points of the PAT, in particular on signals obtained by (PPG), leads to risk of lack of repeatability.

Campo et al (Am J Hypertens. 2017;30(9):876-883) disclosed measurement of arterial stiffness, using a bathroom scale and combining the principles of ballistocardiography (BCG) and impedance plethysmography on a single foot.

Geshe et al (Eur J Appl Physiol. 2012 Jan;112(1):309-15) disclose a function linking systolic blood pressure (SBP) and PWV and its reliability for the determination of absolute SBP. PWV is calculated from the PTT (Pulse Transit Time), which is defined as the time delay between the R-wave of the ECG and the arrival of the pulse wave in the periphery (finger), but the authors approximate the PTT by the PAT measure. They also use the height of the patient and a body correlation factor for approximating the carotid-femoral distance.

Van Velzen et al (J Clin Monit Comput. 2019; 33(2): 241-247) compared pulse wave velocities measured with Complior or Biopac systems (the Complior system measures PWV between the carotid and radial arteries by piezoelectric clips, whereas the the Biopac system uses ECG and optical sensors to measure the PWV between the heart and the fingertips). The PWV measured by the Biopac system was calculated by dividing the distance between the PPG-sensor on the left index finger and the sternoclavicular joint (D) by the time-difference between the R-peak of the ECG and the foot of the pulse wave (i.e. by using the PAT). The authors found that either system could be used to measure PWV changes over time.

Alivon et al (Arch Cardiovasc Dis. 2015;108(4):227-34) propose to measure the PWV using the finger to toe pathway, using two photodiode sensors, positioned on the finger and the toe, recording pulse waves continuously for 20 seconds, and calculating the difference in pulse wave transit time between toe and finger. The distance is estimated using subject height.

The authors of "The Reference Values for Arterial Stiffness' Collaboration" (European Heart Journal, Volume 31, Issue 19, October 2010, Pages 2338-2350) establish reference and normal values for PWV, combining a sizeable European population after standardizing results for different methods of PWV measurement.

Beutel et al (IEEE Transactions on Biomedical Engineering, vol. 68, no. 9, pp. 2810-2820, Sept. 2021) disclose a proof-of-concept for a novel method to estimate central PWV and Blood Pressure.

Huttunen et al (2019, PLOS Computational Biology 15(8): e1007259) studied whether it is possible to estimate cardiovascular health indices using machine learning approaches, in particular by carrying out theoretical assessment of estimating aortic pulse wave velocity, diastolic and systolic blood pressure and stroke volume using pulse transit/arrival timings derived from photopletyshmography signals. They concluded that aortic pulse wave velocity and diastolic blood pressured might be predicted with a high accuracy, but predictions of systolic blood pressure are less accurate

### DISCLOSURE OF THE INVENTION

The present invention provides a simple method for estimating the pulse wave velocity, using values from an electrocardiogram (ECG) and a photo-plethysmograph, which can be combined in a formula to obtain a value for PWV. The inventors showed that the formula should also take the age of the patient into consideration. This method can be repeated over multiple time points, and the PWV can thus be continuously monitored. In particular, the method can be performed in the operating room during all the duration of an anesthesia of a patient during a surgical intervention, using only usual monitoring tools present for monitoring anesthesia. Rather than using the PAT as an approximation of the PTT, the inventors have designed a specific function linking the PWV and the PAT, which also takes the age of the patient into consideration.

The invention thus relates to an *ex vivo* method for evaluating pulse wave velocity (PWV) of a patient and obtain an estimated PWV (PWVest), comprising combining the age of the patient, expressed in years), the carotid-femoral distance (Dcf) of the patient, expressed in meters (m), and the pulse arrival time (PAT), expressed in seconds (s), in a function to obtain an end result,
wherein the function is $\frac{Dcf \left(m\right)}{PAT \left(s\right)} \times \left(k1\times age \left(years\right)+k2\right)$ with
- $0.05 \leq k 1 \leq 0.15$
- $3.5 \leq k 2 \leq 5.0$ wherein the end result provides an estimated PWV expressed in m/s.

In a preferred embodiment, the method is computer-implemented.

In some embodiments, the method also comprises at least one of signal from an ECG from the patient and receiving a signal from a PPG from the patient.

The method may also comprise a step of calculating the PAT (duration between the R-peak of the ECG signal and the foot of the pulse wave of the PPG signal).

Consequently, is herein described an is also a subject of the invention, an *ex vivo* computer-implemented method for evaluating pulse wave velocity of a patient, comprising
- receiving a signal from a ECG from the patient,
- receiving a signal from a PPG from the patient
- calculating the Pulse Arrival Time (PAT), being the duration between the R-peak of the ECG signal and the foot of the pulse wave of the PPG signal
- combining the age of the patient (in years), the carotid-femoral distance (Dcf) of the patient, expressed in meters, and the Pulse Arrival Time (PAT), expressed in seconds, in a function to obtain an end result,
wherein the function is (Dcf (m) / PAT (s)) * (k1 * age (years) + k2), with
- $0.05 \leq k 1 \leq 0.15$
- $3.5 \leq k 2 \leq 5.0$
wherein the end result provides an estimated PWV expressed in m/s.

In one embodiment, the ECG from which the value is obtained has been obtained on D1 and D2 leads. However, an ECG made on any other derivation would also provide the needed signal (peak of the R wave).

In the preferred embodiment, the PPG has been obtained from a plethysmograph placed on a finger of the patient. In other embodiments, the plethysmograph is placed on a patient's toe. In this case, the distance that would be taken into consideration may be adapted and the patient's height can be used, rather than the carotid-femoral distance. The carotid-femoral distance can be measured according to the teachings of the prior art.

The method is performed *ex vivo,* and thus doesn't include the steps of collecting the ECG or PPG data, although it uses such data after receipt of them. It is also noted that the methods herein disclosed provide an estimated value for the PWV, but that it isn't sufficient, by itself, to determine the health status of the patient, as this would require further doctor expertise. This is an intermediary result that will be taken into consideration, by a medical doctor, for posing a diagnosis of a cardiovascular risk, also taking into account the age and other comorbidities (such as weight, body mass index, diabetes, high blood pressure...).

In some embodiments, though, the method may also include the steps of obtaining/measuring the ECG and PPG waves from the patient before transmitting the information for analysis and calculation of the estimated PWV.

In a preferred embodiment, and although the method can be used to calculate a "flash" PWV (i.e. calculate a PWV by using only one Pulse Arrival Time calculated from one ECG signal and the associated PPG signal), it is preferred to obtain the estimated PWV from multiple "flash" measures, which are then averaged. Consequently, in this embodiment, the estimated PWV is calculated as the average of a plurality of estimated PWV calculated over a predetermined period of time. Each of the estimated PWV from this plurality are calculated according to the method disclosed above, using the PAT calculated from one ECG signal and the associated PPG signal.

There are multiple ways to calculate the Pulse Arrival Time. It is reminded that it is the time delay from the R-peak of an electrocardiogram (ECG) waveform to the foot of the pulse wave of a photoplethysmogram (PPG), preferably placed on a patient's finger.

In a first embodiment, the foot of the pulse wave is determined for the maximum of the second derivative, with respect to time, of the photoplethysmographic signal at the rising edge of the pulse wave. This method is used in the art and disclosed, in particular, in Chiu et al. (Am Heart J. 1991. 121, 1460) or in US20200390347.

In particular, the foot of the pulse wave is determined by calculating the second derivative of the plethysmographic signal, the foot of the pulse wave corresponding to the maximum of this second derivative at the rising edge of the pulse wave. The first derivative can be weighted to focus only on the rising part of the signal (i.e. the second derivative is given a zero value when the first derivative is not positive). Alternatively, the values obtained can be squared and then integrated over floating windows of predetermined time, in particular 240 ms centered (averaged over the values 120 ms before and 120 ms after the desired point), in order to obtain a strong signal at each rising edge of the pulse wave. This signal can be compared with a threshold value. Preferably, this threshold value is adaptive in real time. The threshold value can be calculated by the formula: the integral (as calculated above) averaged over a floating window of 3 s (centered or not), and whose value is multiplied by 1.5. The peak (maximum) of the second derivative (which defines the foot of the wave) is identified in the area where the integral exceeds this threshold value.

In another embodiment, the foot of the pulse wave is determined by the method of the intersecting tangents. In this method, the point of intersection of the tangents for (1) the point at the minimum of the pulse wave (which is an horizontal line) and (2) the point corresponding to the maximum of the first derivative (with respect to time) of the photoplethysmographic signal at the rising edge of the pulse wave. This method is used in the art and disclosed, in particular, in Chiu et al. (*op. cit*.).

As indicated in Chiu et al (*op. cit*.), these two methods were the most consistent and widely applicable methods for the determination of PWVs. Other methods may also be contemplated for determining the foot of the pulse wave of the PPG, such as using the point of minimum diastolic pressure, or the point at which the first derivative of pressure is maximum (corresponding to the rise of the pulse wave).

Using the time point corresponding to the R-peak and the time point corresponding to the foot of the pulse wave, the Pulse Arrival Time is defined as the duration between these two time points (for the same heart beat). The time points (moment) of the R-peak and foot of the pulse wave can be determined by a timekeeping unit such as an internal clock.

As indicated above, the estimated PWV (PWVest) can be obtained by the formula: $PWVest \left(m/s\right) = \frac{Dcf \left(m\right)}{PAT \left(s\right)} \times \left(k1\times age \left(years\right)+k2\right)$ with
- 0.05 ≤ k1 ≤ 0.15, preferably 0.05 ≤ k1 ≤ 0.10, preferably 0.06 ≤ k1 ≤ 0.08, more preferably 0.07 ≤ k1 ≤ 0.086, more preferably 0.075 ≤ k1 ≤ 0.080
- 3.5 ≤ k2 ≤ 5.0, preferably 3.6 ≤ k2 ≤ 4.6, preferably 3.85 ≤ k2 ≤ 4.40, more preferably 4.00 ≤ k2 ≤ 4.25.

In a specific embodiment, k1 equals about or exactly 0.079 and k2 equals about or exactly 4.12 (about meaning the value ± 2%).

As indicated above an average PWV can be estimated by estimating multiple flash PWV over a certain period of time and averaging these values.

It is also possible to repeat the method herein disclosed at different times t to estimate multiple PWV for the patient. This makes it possible to draw a curve of estimated PWV in function of the time. When the blood pressure (in particular the mean arterial pressure) is also known in function of the time, it is the possible to compare the variation of the estimated PWV in function of the blood pressure.

The different estimations of PWV can be "flash" PWV estimations or estimation of multiple average PWV at multiple time points. As an illustration, is the duration is twelve heart beats, one can obtain twelve estimated "flash" PWV values, or, if an average PWV is calculated for three heart beats, one will obtain four average PWV values.

The method can advantageously be carried out to estimate the PWV of an anesthetized patient, during anesthesia of the patient (notably a general anesthesia). This is of particular advantage as (1) the method makes use of a ECG signal and of a PPG signal, which are obtained by devices that are already used in operating rooms for monitoring anesthesia, (2) being anesthetized, the patient is in an essentially constant state (with the proviso that there may be variation of the mean arterial pressure), thereby increasing the reliability of the results, (3) the arterial pressure being regularly (through a cuff, i.e. an oscillometric brachial tensiometer) or continuously monitored (in particular according to a method of US 20200390347, notably by using the height of the dicrotic wave height), it is possible to link the estimated PWV at a given time point with the mean arterial pressure at that time point, thereby expressing the PWV ad a function of the mean arterial pressure and (4), in view of the duration of the anesthesia, it is possible to repeat the method and obtain multiple PWV values, thereby providing interesting information to the physician (such as highest estimated PWV, lowest estimated PWV, mean PWV over the whole duration of data gathering, median PWV over the whole duration of data gathering, standard deviation...).

The method herein disclosed can be performed by a device for estimating the pulse wave velocity in a patient, comprising
a. means for receiving an electrocardiogram (ECG) signal and a photoplethysmography signal from the patient
b. means for determining the R-peak of the ECG signal
c. means for determining the foot of the pulse wave signal
d. means for calculating the Pulse Arrival Time (PAT)
e. means for calculating an estimated Pulse Wave Velocity (PWV), by combining the age of the patient, the carotid-femoral distance (Dcf) of the patient and the calculated Pulse Arrival Time (PAT) in a function to obtain an end result,
   wherein the function is (Dcf (m) / PAT (s)) * (k1 * age (years) + k2), with
      i. $0.05 \leq k 1 \leq 0.15$
      ii. $3.5 \leq k 2 \leq 5.0$
   wherein the end result provides an estimated PWV expressed in m/s,
f. means for recording/storing the estimated PWV in a database and preferably also recording/storing the time point (the moment) on which it has been calculated. This makes it possible to later retrieve these values for further analysis.

The device may also include a storage unit / database including the age and the carotid-femoral distance (that was previously measured) for the patient. The carotid-femoral distance can be measured as known for the ultrasound and tonometry techniques. Alternatively, it can be determined as being half the height of the patient, as there is highly significant correlation between arm span and body height.

The means for receiving the electrocardiogram (ECG) signal and the photoplethysmography (PPG) signal from the patient may comprise an analog-to-digital converting unit, which converts an analog electrocardiogram (or PPG) signal into a corresponding digital electrocardiogram (or PPG) signal. Such types of units are disclosed in particular in US8285369.

The means for a. means for determining the R-peak of the ECG signal are electronically linked to the means for receiving the electrocardiogram (ECG) signal and comprise a storage unit and a digital signal processor. The digital ECG signal is stored within the storage unit, together with the associated times of the wave form, and the point R-peak of the ECG is determined by the signal processing unit as the highest value of the ECG signal. The time corresponding to this R-peak is stored in a storage unit.

The means for determining the foot of the pulse wave signal are electronically linked to the means for receiving the PPG signal and comprise a storage unit and a digital signal processor. The digital PPG signal is stored in the storage unit, together with the associated times of the wave form, and the point corresponding to the foot of the pulse wave is determined according to the methods mentioned above. The time corresponding to the foot of the pulse wave is stored in the storage unit.

The means for calculating the Pulse Arrival Time (PAT) include a processing unit that retrieves the times of the R-peak and of the foot of the pulse wave and that makes the difference between these times so that PAT = t(foot of the pulse wave) - t(R-peak). The PAT value is stored in a database.

The means for calculating an estimated PWV include a processing unit that retrieves the PAT, the age of the patient and the carotid-femoral distance (Dcf), and combines these values through the function disclosed above. In some embodiments, the means for calculating the estimated PWV retrieves multiple PAT successively stored in the database, performs an average of these multiples PAT to obtain an average PAT value that is stored in a database (preferably together with an associated time). In other embodiments, the means for calculating the estimated PWV further comprises a processing unit that calculates an average of multiple PWV (in particular a rolling average) and stores the results in a database.

The end result may then be stored in a database (or a storing unit), preferably with a time thereto (such as the time (the moment) on which it has been calculated, or the time of the R-peak, or the time of the foot of the pulse wave...). This makes it possible to later retrieve these values for further analysis (so as to calculate the mean, median, highest value, lowest value, standard deviation...) of the various estimated PWV.

The device may also comprise means for displaying the estimated PWV on a monitor. It is preferred when the data stored in the storage units or the databases of the device can be exported, for further analysis, in particular by a physician.

In some embodiments, a processing unit calculates the mean, median, highest value, lowest value, standard deviation of the plurality of PWVest stored in the database. Such calculated results can be stored in an appropriate database (that would also include an ID of the patient), displayed on a monitor, exported or sent to a remote terminal.

The device may also comprise means for estimating the Mean Arterial Pressure (MAP) and obtain an estimated Mean Arterial Pressure (MAPest). Such devices are disclosed in US 20200390347. They include a processing unit that calculates the foot of the pulse wave (as disclosed above), the peak (maximum) of the pulse wave, and the value (height) and time of the dicrotic wave.

As explained in US 20200390347, the peak of the pulse wave can be determined, after the foot of the pulse wave by (i) splitting the signal into several time windows (in particular windows between 20 ms and 100 ms, in particular 50 ms), and (ii) looking at the maximum value in each time window (one advances window by window as long as higher values are found in the last time window studied). The local maximum value (corresponding to the maximum value V found in a time window, the maximum value in the next time window being less than this value V) corresponds to the maximum of the pulse wave. This makes it possible to obtain the value of the maximum (absorbance value given by the pulse oximeter corresponding to the peak of the systolic wave) and the time at which this maximum is reached.

As explained in US 20200390347, The value and time of the dicrotic wave can be determined by (i) obtaining the second derivative of the signal after the pulse wave maximum, (ii) splitting the signal over predetermined time windows (between 50 ms and 300 ms, in particular 150 ms) after this pulse wave peak, (iii) searching for the local maximum of the second derivative of the signal in each time window, in order to determine an area of interest (time window in which this local maximum of the second derivative of the signal is present) and (iv) searching for the absolute minimum value of the first derivative of the signal in this area of interest. This search can also be performed by splitting the area of interest into time windows (between 5 ms and 15 ms, in particular 8 ms). The point corresponding to the dicrotic wave corresponds to the point at which the absolute minimum value of the first derivative is reached. Its value (absorbance value given by the pulse oximeter) as well as the time between the foot of the pulse wave and this point can then be measured and determined.

A processing unit can estimate the MAP for to this pulse wave, by the formula MAPest = Calib x Vpt, wherein Calib = (Mean Arterial Pressure measured at a predetermined time, for instance at a time where the Arterial Pressure has been measured by the cuff) / (height of the dicrotic wave at this predetermined time).

The estimated MAPest can be stored in a database in a storing unit, as well as the time where it has been estimated.

The device may also comprise means for storing, displaying, exporting or sending the plurality of estimated PWV and MAP at different times.

The device may also comprise means for calculating the ratio (estimated PWV) / (estimated MAP) (expressed for instance in m.s⁻¹ / mmHg), which represents an index of the arterial distensibility.

Consequently, the device specially intended for preforming the methods herein disclosed makes it possible to obtain an estimated value for the PWV (and for the MAP, if applicable) for each heartbeat (for each ECG and pulse wave). This device makes it possible to perform these methods continuously, and obtain a large set of values that will provide better information to the physician that will ultimately make the prognosis with regards to the cardiovascular risk for the patient. When the MAP is also continuously evaluated, such information will allow the physician to ponder the information pertaining to the PWV alone with the MAP, which will allow a more accurate diagnosis.

A database that is implemented during performance of the methods described above can be organized as to contain, for a heartbeat, a table containing, as data
- The digital image of the ECG wave for the heartbeat
- The digital image of the pulse wave of the heartbeat
- The time (hour, minute, second) of the R peak of the ECG wave
- The time (hour, minute, second) of the foot of the pulse wave
- The Pulse Arrival Time as calculated for the heartbeat
- The Pulse Wave Velocity as calculated for the heartbeat

This table shall be in relation with another table comprising identification information for the patient (for instance name, surname identification number, birthday...), the carotid-femoral distance of the patient and the age of the patient. Such relation is needed for retrieving the information necessary for calculation of the PWV.

The invention also pertains to a computer product/program comprising program code instructions recorded on a computer-readable medium, for carrying out the steps of the methods herein disclosed, when said program is executed on a computer, as well as a computer-readable recording medium on which is recorded a computer program comprising program code instructions for performing the steps of the methods herein disclosed.

The invention also pertains to a computer program product comprising instructions to perform the methods herein disclosed, when such instructions are executed by a logical circuit or a processor.

The invention also relates to a non-transitory computer-readable storage medium comprising program instructions for causing a computing apparatus to execute consecutive steps of a method for estimating Pulse Wave Velocity (PWV) of a patient, and optionally MAP of the patient by implementing the methods herein disclosed. In particular, the method comprises:
- Receiving an electrocardiogram (ECG) signal
- Receiving a photo-plethysmography (PPG) signal
- Converting the ECG and PPG signal into digital signals
- Identifying the R-peak and its time in the digitalized ECG signal and the foot of the pulse wave and its time in the PPG signal
- Calculating the Pulse Arrival Time (PAT)
- Obtaining the age and the carotid-femoral distance (Dcf) of the patient
- Estimating the PWV of the patient according to the formulas herein disclosed.

The disclosure also relates to a method, which is not claimed, for treating a subject, comprising performing the methods herein disclosed to evaluate the PWV, in particular during anesthesia of the subject and treating the subject with a cardiovascular drug (in particular an anti-hypertension drug) when the evaluated PWV is high. Such treatment may comprise starting administration of the cardiovascular drug, or modifying/adapting the treatment in case the subject already has cardiovascular treatment.
it is to be noted that the PWV increases with age of the subject, as well as with the presence of pre-existing clinical condition. Koivistoinen et al (Clin Physiol Funct Imaging (2007) 27, pp191-196) have shown that normal values depend on the age and of the sex of the subject, when the subject has no sign of a cardiovascular disease.

**Table 1. From Koivistoinen et al (op. cit.)**

| Age (years) | Male (PWV ± SD) | n | Female (PWV ± SD) | n | P-value |
|---|---|---|---|---|---|
| 26-41 | 7.7 ± 1.2 | 47 | 7.0 ± 1.0 | 71 | 0.002 |
| 42-59 | 9.1 ± 1.5 | 57 | 8.2 ± 1.6 | 59 | 0.005 |
| 60-75 | 10.7 ± 1.7 | 26 | 10.4 ± 2.9 | 23 | 0.712 |
| All | 8.9 ± 1.8 | 130 | 8.1 ± 2.0 | 153 | <0.001 |

Diaz et al (Int J Hypertens. 2014; 2014: 653239) have provided reference values of Pulse Wave Velocity in healthy people from an urban and rural Argentinean population and have found the following values.

**Table 2. From Diaz et al (op. cit.)**

| Age group (years) | n | Mean PWV (m/s) | SD | 95% CI Lower-upper limit | Range |
|---|---|---|---|---|---|
| 10-19 | 156 | 5.04 | 0.72 | 4.92-5.15 | 3.12-7.33 |
| 20-29 | 110 | 5.86 | 0.92 | 5.68-6.03 | 3.92-8.14 |
| 30-39 | 109 | 6.32 | 0.82 | 6.16-6.47 | 4.08-8.26 |
| 40-49 | 108 | 6.85 | 0.91 | 6.68-7.03 | 5.0-9.84 |
| 50-59 | 164 | 8.15 | 1.17 | 7.97-8.33 | 5.46-12.5 |
| 60-69 | 103 | 8.47 | 1.09 | 8.25-8.68 | 6.46-11.2 |
| >70 | 30 | 9.01 | 2.00 | 8.27-9.76 | 5.52-13.4 |
| Total | 780 | 6.84 | 1.65 | 6.73-6.96 | 3.12-13.4 |

Thus, for a subject with no evidence of a cardiovascular disease, an estimated PWV higher than 10 m/s (in particular for a patient aged 59 or less) would trigger the need for further advice for determining the presence of a cardiovascular disease and/or treatment with a cardiovascular drug. The threshold may be 12 m/s for subjects aged 60 or more.

For dialysis patients, Ferreira et al (Am J Nephrol. 2017;45(1):72-81) have shown that a PWV >12 m/s provides important prognostic information of cardiovascular mortality in patients with end-stage renal disease under 60 years of age. Using the method herein disclosed would lead to treatment of such patients with an adequate cardiovascular drug, and notably a drug for managing hypertension.

A number of drug classes are effective for initial and subsequent management of hypertension and can be administered to subject for which a high PWV has been evaluated according to the methods herein disclosed. One can cite adrenergic modifiers (such as central alpha-2-agonists (notably methyldopa, clonidine, guanabenz, guanfacine) or postsynaptic alpha-1-blockers (such as prazosin, terazosin, doxazosin)), angiotensin-converting enzyme (ACE) inhibitors (such as Benazepril, Captopril, Enalapril, Fosinopril, Lisinopril, Perindopril erbumine, Quinapril, Ramipril, Trandolapril), angiotensin II receptor blockers (ARBs) (such as Azilsartan, Candesartan, Eprosartan, Irbesartan, Losartan, Olmesartan, Telmisartan, Valsartan), beta-blockers (such as Acebutolol, Atenolol, Betaxolol, Bisoprolol, Carvedilol (including controlled-release), Labetalol, Metoprolol (including extended-release), Nadolol, Nebivolol, Penbutolol, Pindolol, Propranolol, Timolol), calcium channel blockers such as Dihydropyridines (Amlodipine, Felodipine, Isradipine, Nicardipine (including sustained-release), Nifedipine (notably extended-release), Nisoldipine), direct renin inhibitors (in particular Aliskiren), direct vasodilators (including minoxidil and hydralazine) or diuretics (Loop diuretics, Potassium-sparing diuretics, Thiazide-type diuretics).

The methods herein disclosed may also be of use for administering the proper drug to a subject during an anesthesia, especially when the subject has hypotension during the anesthesia. Indeed, when a high PWV is evaluated, using the methods herein disclosed, the physician in charge of the anesthesia will administer not only pure vasopressors as phenylephrine or norepinephrine. They could use an association with vasopressor and an inotrope or chronotrop drugs (such as a positive inotropic agent (notably Digoxin, Berberine, Calcium, Calcium sensitisers, Levosimendan, Catecholamines, Dopamine, Dobutamine, Dopexamine, Adrenaline (epinephrine), Isoproterenol (isoprenaline), Eicosanoids, Prostaglandins, Phosphodiesterase inhibitors, Enoximone, Milrinone, Amrinone, Theophylline, Glucagon, Insulin)).

On this other way, when the subject has hypotension, but has a PWV that is not too high, the physician may safely use a vasopressor alone (such as norepinephrine or phenylephrine) because the increase in vascular tone induced by the vasopressor will not increase PWV in such a way that it could become dangerous for the subject.

Hence, estimating the PWV value during hypotension episode can help the physician of restoring hemodynamics by choosing the right drugs at the right time.

### DESCRIPTION OF THE FIGURES

Figure 1: Relation between PAT/TTm ratio and age. Y = 0.07868X + 4.124. r=0.8089.
R²=0.6544. P<0.0001.
Figure 2: Relation between LAAsc and PAT/TTm ratio. Y = 0.8743X + 1.842.
r=0.8089. R²=0.6544. P<0.0001
Figure 3: Evolution of the PAT/TTm ratio according to age. Left: less than 50 years old. Middle: between 50 and 75 years old. Right: more than 75 years old
Figure 4: Relation between carotid-femoral TT and TTlrb. r=0.7942. R²=0.6307. p<0.0001
Figure 5: Mean of the measured PWVcf vs the estimated PWV-Irb.
Figure 6: Relation between PWV and PWV-Irb. r=0.7427. R²=0.5516. p<0.0001
Figure 7: Brand-Altman representation between PWVcf and PWVlrb
Figure 8: Variation PWVcf/MAP or PWVlrb/MAP for three patients (young, intermediate, aged). Circles: young patients with few PWV variations generated by PAM variations (Black: PWVcf; grey: PWVlrb). Diamonds: intermediate patients with strong variations of PWV with variations of PAM (Black: PWVcf; grey: PWVlrb). Triangles: old patients with few variations of PWV with variations of PAM but with high values of PWV compared to the population (Black: PWVcf; grey: PWVlrb).

### EXAMPLES

The examples below illustrate different aspects and modes of implementation of the invention. The embodiments described in the examples are an integral part of the invention.

### Example 1. Principles

During each anesthesia, in order to detect cardiovascular risk factors, it was sought to measure PWV (carotid-femoral) non-invasively and continuously. The aim was to develop a method that would estimate the PWV continuously in the operating room during the entire duration of an intervention, using only usual monitoring tools, namely: an electrocardiogram (ECG), a digital photoplethysmography (PPG) and, further with the use of an oscillometric brachial blood pressure monitor to measure the blood pressure in a non-invasive way (NIBP). Moreover, the hemodynamic variations inherent to anesthesia make it possivle to explore the PWVcf on a large panel of arterial pressure variations, but also to obtain a true arterial distensibility index expressed in m/s per mmHg.

Thus, from a simple, automated and extended access, the continuous measurement of PWV in the operating room during each general anesthesia, taking into account the variations of Mean Arterial Pressure (MAP) would solve the limitations described in introduction, and could thus have a considerable impact in terms of screening and follow-up of patients at cardiovascular risk.

The work was focused on the measurement of a time: Pulse Arrival Time (PAT) (time between the R wave of the ECG and the foot of the digital PPG wave corresponding to the "a" wave of the second derivative of the digital PPG signal). This parameter can be continuously monitored in the operating room.

From a physiological point of view, the PAT is the sum of two durations :
- the Pre Ejection Period (PEP): corresponding to the phase of the isovolumetric contraction before ventricular ejection
- the Pulse Transit Time (PTT): corresponding to the transit time of the pulse wave between the opening of the aortic valve and the foot of the digital PPG wave signal measured with the finger.

So that PAT = PEP + PTT.

Thus with a digital PPG, the PAT is the sum of the times related to the PEP and the transit time in the ascending aorta, then in the subclavian artery then humeral to the digital artery. It can be approximated as the length between aorta and carotid.

The aim was to provide a formula that would link the measurement of PAT to the PWV. This was achieved using a patient's cohort (patients were anesthetized, as they had to have surgical operations for different reasons), and it was found that it is possible to express PWV in function of the PAT, the age and the carotid-femoral distance of the patient (Dcf). $PWVest \left(m/s\right) = \frac{Dcf \left(m\right)}{PAT \left(s\right)} \times \left(k1\times age \left(years\right)+k2\right)$

With
- 0.05 ≤ k1 ≤ 0.15, preferably 0.05 ≤ k1 ≤ 0.10, preferably 0.06 ≤ k1 ≤ 0.08, more preferably 0.07 ≤ k1 ≤ 0.086, more preferably 0.075 ≤ k1 ≤ 0.080
- 3.5 ≤ k2 ≤ 5.0, preferably 3.6 ≤ k2 ≤ 4.6, preferably 3.85 ≤ k2 ≤ 4.40, more preferably 4.00 ≤ k2 ≤ 4.25.

In a specific embodiment, k1 equals about or exactly 0.079 and k2 equals about or exactly 4.12 (about meaning the value ± 2%)

Determination of the values of k1 and k2 was achieved by:
Step 1: Almost simultaneous measurements at different blood pressure levels and for several patients of PAT and PWVcf (using gold stantard method). This made it possible to obtain the transit time TT because PWVcf = Dcf / TT where Dcf is the carotid-femoral distance).

It was observed that, for each patient, the PAT moves in the same direction as TT.

Step 2: Calculation of the PAT ratio, for each patient, for various levels of blood pressures.

It was found that the PAT/TT ratio was not very dependent on blood pressure values.

Step 3: the mean ratio PAT/TTm was calculated for each patient, corresponding to the mean of the PAT/TT ratios at different levels of blood pressures.

As indicated above, PAT = PEP + PTT with PTT corresponding to the transit time in the descending aorta, the subclavian, humeral and digital arteries.

Step 4: Estimation of the length of the ascending aorta (LAAsc) for each cohort patient, with the Sugarawa formula.

Sugawara et al (JACC Cardiovasc Imaging 1, 739-748, 2008) showed that the ascending aorta lengthened significantly with aging without this association being found with the other aortic segments (descending thoracic aorta and abdominal aorta). They estimated the length of the ascending aorta (LAAsc) with the following formula: LAAsc (cm)= 0.09 x age +2.61

Step 5: Analysis of the relation between PAT/TTm ratio and LAAsc for each patient. A strong linear relation was found between PAT/TTm and LAAsc *via* the following formula: PAT/TTm=0.8743 x LAAsc +1.842

Thus, the carotid-femoral transit time (designated as TTlrb) can be estimated, from the length of the ascending aorta and the PAT, as:

TTlrb = PAT / (0.8743 x LAAsc +1.842).

Step 6: Estimation of the carotid femoral PWV (PWVcf or PWVlrb) from the preceding results. $PWVIrb \left(m/sec\right) = Dcf / \left(TTIrb\right)$ $PWVIrb \left(m/sec\right) = Dcf / \left[PAT/\left(0.8743\times \left(0.09\times âge+2.61\right)+1.842\right)\right]$ $PWVIrb \left(m/sec\right) = Dcf / \left[PAT/\left(0.078687\times age+2.281923+1.842\right)\right]$ $PWVIrb \left(en m/s=\left(Dcf/PAT\right)\times \left(0.078687\times age +4.123923\right)\right.$ $PWVIrb \left(en m/s=Dcf/PATx\left(k1x age+k2\right)\right.$ with Dcf expressed in m and TTlrb expressed in sec (s) and age expressed in years. In this cohort, k1 = 0.078687 and k2=4.123923. It can be rounded to k1=0.079 and k2=4.12. The coefficients herein disclosed may slightly vary, depending on the size of the cohort,

### Conclusion

The completion of the two phases of the work allowed concluding that the formula makes it possible to estimate the carotid-femoral pulse wave velocity in a simple way from the age, the length of the aorta (approximated from the carotid-femoral distance, or the shoulder-palm of the hand distance) and the measurement of the PAT. Thus, the PWVlrb (PWVest) measurement in the operating room in patients under general anesthesia appears to have the potential to solve the limitations associated with PWVcf measurement in cardiovascular risk screening:
- by the simplicity of PWVlrb measurement: no specific equipment except ECG, digital photoplethysmography (PPG) and oscillometric brachial tensiometer) therefore inexpensive compared to other methods, nor specific training.
- by the ability to continuously measure and estimate the PWVlrb and to combine it with the punctual measurements of arterial pressures, allowing the calculation of PWVlrb/PAM variations, that is to say a dynamic and personalized analysis of the arterial stiffness of patients.

### Example 2. Application of the principles

### Methods:

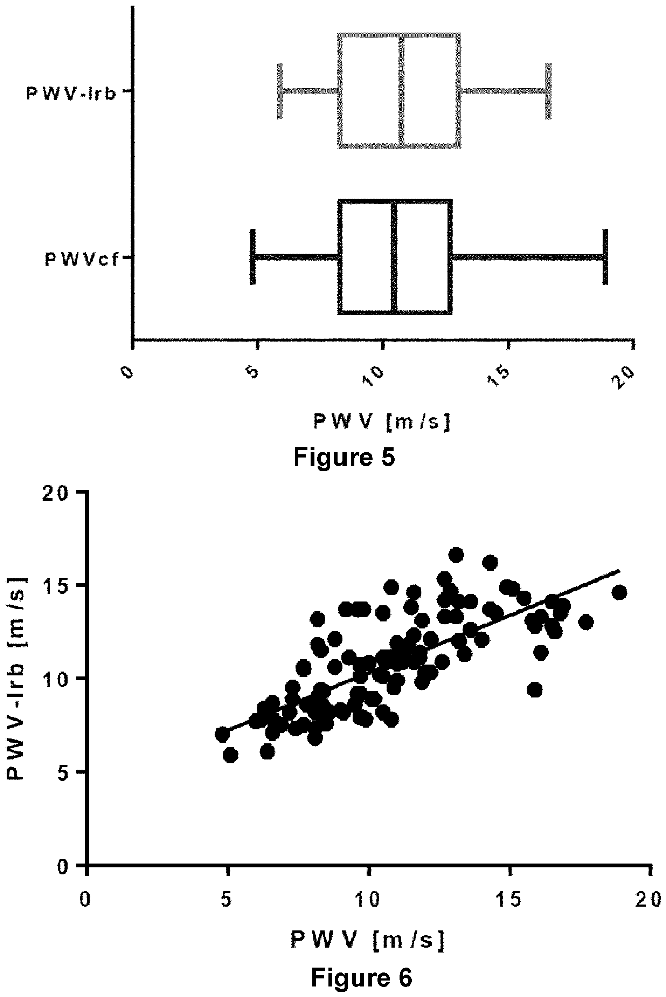

The study was conducted in the operating room on adult patients undergoing anesthesia (general or spinal). A standard monitoring system consisting of an ECG, a digital PPG and a blood pressure cuff was installed. In addition to this monitoring, a carotid arterial tonometer and a thigh cuff (SphygmoCor XCEL^{®}, AtCor Medical) were used to measure the PWVcf on the side homolateral to the digital PPG signal. PWVcf measurements were performed occasionally: one in the basal state (before anesthesia) and several under anesthesia (1 to 3 measurements per patient). When a PWVcf measurement was initiated, a concomitant blood pressure cuff measurement was performed. Continuous recording of intraoperative parameters (ECG, digital PPG, BP) was performed using iXtrend^{®} software. The analysis of the raw ECG and digital PPG signal allowing the measurement of the PAT was performed with the Graphysio software. The PAT corresponded to the time between the peak of the R wave on the ECG and the peak of the "a" wave of the second derivative of the digital PPG signal (Figure 5). PAT concomitant with a PWVcf measurement was averaged over 4 beats.

### Results:

44 patients beneficiated from at least ont PWVcf measure associated with a PAT measure, with a toal of 118 PWVcf - PAT measured pairs.

The PAT/TTm was, on average, 8.734 [8.073-9.395].

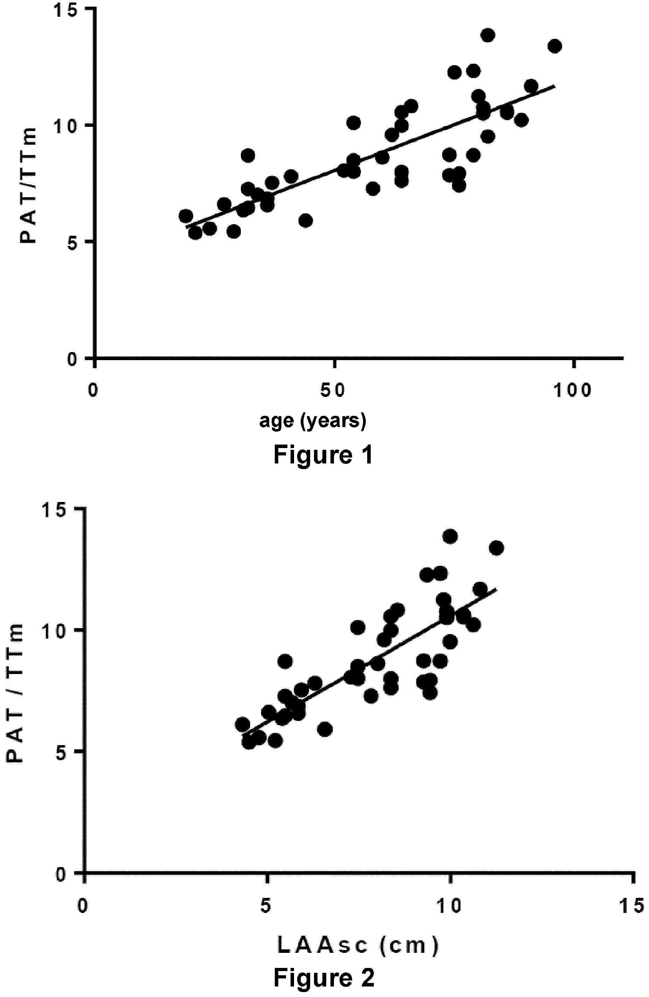

The PAT/TTm was strongly correlated with the age and the ascending aorta length (r=0.8089) (Figures 1 and 2).

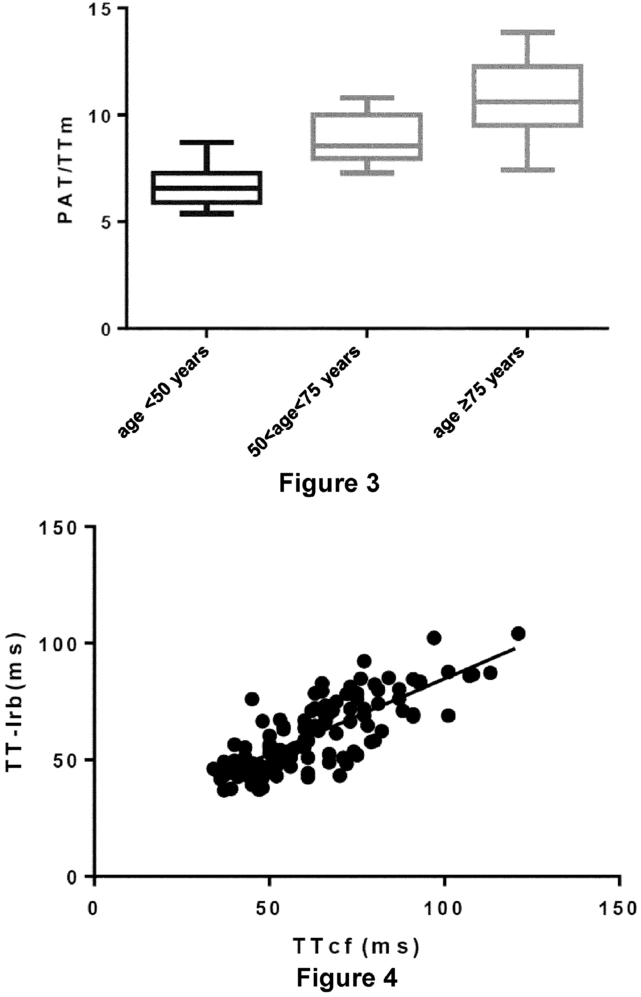

Patients aged below 50 years presented a PAT/TTm ratio of 6.64±0.93, those aged between 50-75 years had one at 8.83±1.16 and those aged 75 years or more presented one at 10.73±1.84 (Figure 3).

TTlrb was highly correlated to the TTcf measured with the le Sphygmocor (r=0.7942) (Figure 4).

The mean of all the measured PWVcf was 10.66±3.065 and the mean of the PWVlrb estimated by the method was 10.74±2.536 (Figure 5).

A high correlation was found between PWV-Irb and PWVcf (r=0.7427) (Figure 6).

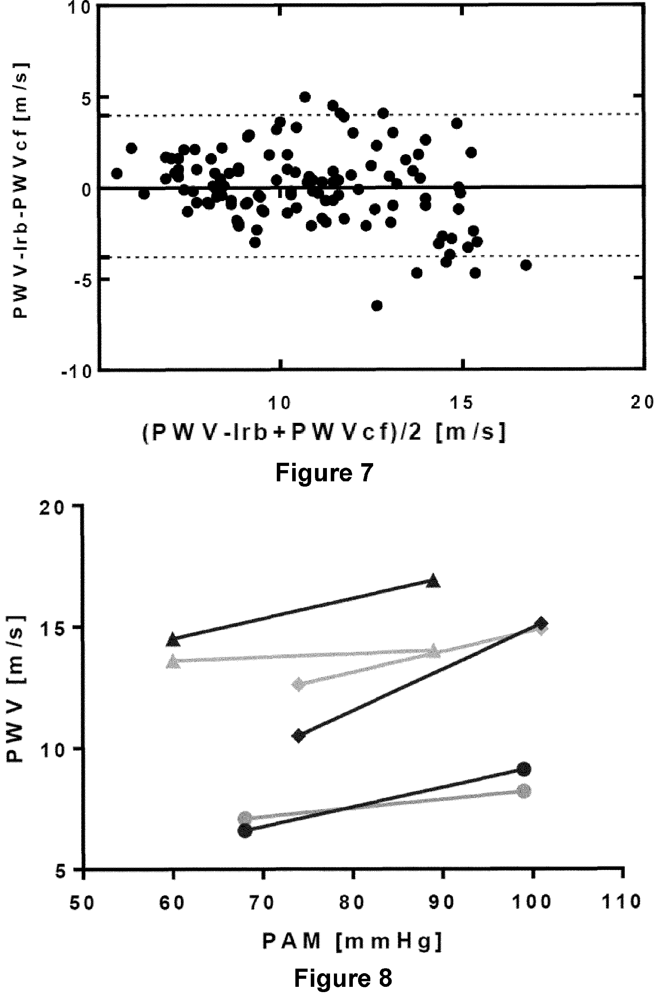

Figure 7 shows that the measures are in agreement *via* a Bland-Altman representation: bias was 0.04 ± 2.07 m/s, whereas the agreement limit at 95% was [-4.012;4.098].

Furthermore, a cross-correlation was performed on this cohort by separating it into two and calculating constants k1 and k2 for one half of the cohort and validating on the other half. The results are also similar between the PWVcf and PWVlrb.

Finally, the results were modeled by plotting the slope of the line between variations in blood pressure and variations in PWV (visualization of the PWV/PAM ratio or ΔPWV/ΔPAM).

This modeling finds three types of population characterized by three examples (Figure 8):
(1) young patients with few PWV variations generated by PAM variations.
(2) intermediate patients with strong variations of PWV with variations of PAM and finally
(3) very old patients with few variations of PWV with variations of PAM but with high values of PWV compared to the population (1).

With the PWVlrb, it is possible to perform this type of modeling continuously in the operating room by following the variations of MAP induced by anesthesia.

### Conclusion

This work shows that from a classical monitoring during anesthesia (ECG, digital PPG, blood pressure cuff) and with a simple additional anthropometric measurement (carotid-femoral distance), an estimation of PWVcf can be obtained, as the calculated PWVlrb

## Claims

1. An *ex vivo* computer-implemented method for evaluating Pulse Wave Velocity (PWV) of a patient, comprising combining the age of the patient, the carotid-femoral distance (Dcf) of the patient, expressed in meters, and the Pulse Arrival Time (PAT), expressed in seconds, in a function PWVest to obtain an end result,
wherein the function is $PWVest \left(Dcf; PAT; age\right) = \frac{Dcf \left(m\right)}{PAT \left(s\right)} \times \left(k1\times age \left(years\right)+k2\right)$ with
- $0.05 \leq k 1 \leq 0.15$
- $3.5 \leq k 2 \leq 5.0$
wherein the end result provides an estimated PWV expressed in m/s.

2. The *ex vivo* method of claim 1, wherein the estimated PWV is calculated as the average of a plurality of estimated PWV calculated over a predetermined period of time.

3. The *ex vivo* method as claimed in one of claims 1 to 2, wherein 0.07 ≤ k1 ≤ 0.086.

4. The *ex vivo* method as claimed in one of claims 1 to 3, wherein 4.00 ≤ k2 ≤ 4.25.

5. The *ex vivo* method as claimed in one of claims 1 to 4, wherein k1 = 0.079 and k2 = 4.12.

6. The *ex vivo* method as claimed in one of claims 1 to 5, which is repeated at different times t to estimate multiple PWV for the patient.

7. The *ex vivo* method as claimed in one of claims 1 to 6, wherein the Mean Arterial Pressure of the patient is also measured or estimated.

8. The *ex vivo* method as claimed in one of claims 1 to 7, which is carried out during anesthesia of the patient.

9. A device for continuously evaluating the pulse wave velocity in a patient, comprising
a. means for receiving an electrocardiogram (ECG) signal and an photoplethysmography signal from the patient
b. means for determining the R-peak of the ECG signal
c. means for determining the foot of the pulse wave signal
d. means for calculating the Pulse Arrival Time (PAT) being the duration between the R-peak of the ECG signal and the foot of the pulse wave of the PPG signal
e. means for calculating an estimated Pulse Wave Velocity (PWV), by combining the age of the patient, the carotid-femoral distance (Dcf) of the patient and the calculated Pulse Arrival Time (PAT) in a function PWVest to obtain an end result,
wherein the function is PWVest (Dcf; PAT; age) = (Dcf (m) / [PAT (s)) * (k1 * age (years) + k2), with
i. $0.05 \leq k 1 \leq 0.15$
ii. $3.5 \leq k 2 \leq 5.0$
wherein the end result provides an estimated PWV expressed in m/s,
f. means for storing the estimated PWV in a database.

10. The device of claim 9, further comprising means for estimating or measuring the Mean Arterial Pressure and means for storing the estimated MAPest and the time it has been measured or estimated.

11. The device of claim 10, which comprises means for calculating the height of the dicrotic wave (Vpt) at time t, wherein the Mean Arterial Pressure (MAPest) at time t is estimated by formula MAPest = Calib x Vpt, wherein Calib = (Mean Arterial Pressure measured at a predetermined time) / (height of the dicrotic wave at the predetermined time).

12. The device of any one of claims 9 to 11, further comprising means for displaying or exporting the estimated PWV.

13. The device of any one of claims 9 to 12, further comprising means for calculating an average PWV by calculating the mean of PWV estimated at different moments.

14. The device of claim 13, further comprising means for displaying or exporting the average PWV, and/or the lowest estimated PWV and/or the highest estimated PWV.

15. A computer program product comprising instructions to perform the method according to any one of claims 1 to 8, when said program is executed on a computer and such instructions are executed by a logical circuit or a processor.

## Patentansprüche

1. E*x vivo* computergestütztes Verfahren zur Bewertung der Pulswellengeschwindigkeit (PWV) eines Patienten, umfassend das Kombinieren des Alters des Patienten, der Karotis-Femoralis-Distanz (Dcf) des Patienten, ausgedrückt in Metern, und der Pulsankunftszeit (PAT), ausgedrückt in Sekunden, in einer Funktion PWVest, um ein Endergebnis zu erhalten,
wobei die Funktion wie folgt lautet $PWVest \left(Dcf; PAT; Alter\right) = \frac{Dcf \left(m\right)}{PAT \left(s\right)} \times \left(k1\times age \left(Jahre\right)+k2\right)$ mit
- $0.05 \leq k 1 \leq 0.15$
- $3.5 \leq k 2 \leq 5.0$
wobei das Endergebnis eine geschätzte PWV liefert, ausgedrückt in m/s.

2. *Ex vivo*-Verfahren nach Anspruch 1, wobei die geschätzte PWV als Mittelwert einer Vielzahl von geschätzten PWV berechnet wird, die über einen vorbestimmten Zeitraum ermittelt wurden.

3. *Ex vivo*-Verfahren nach einem der Ansprüche 1 bis 2, wobei 0,07 ≤ k1 ≤ 0,086 gilt.

4. *Ex vivo*-Verfahren nach einem der Ansprüche 1 bis 3, wobei 4,00 ≤ k2 ≤ 4,25 gilt.

5. *Ex vivo*-Verfahren nach einem der Ansprüche 1 bis 4, wobei k1 = 0,079 und k2 = 4,12 ist.

6. *Ex vivo*-Verfahren nach einem der Ansprüche 1 bis 5, das zu verschiedenen Zeitpunkten t wiederholt wird, um mehrere PWV-Werte für den Patienten zu schätzen.

7. *Ex vivo*-Verfahren nach einem der Ansprüche 1 bis 6, wobei der mittlere arterielle Druck des Patienten ebenfalls gemessen oder geschätzt wird.

8. *Ex vivo*-Verfahren nach einem der Ansprüche 1 bis 7, das während der Anästhesie des Patienten durchgeführt wird.

9. Vorrichtung zur kontinuierlichen Auswertung der Pulswellengeschwindigkeit bei einem Patienten, umfassend
a. Mittel zum Empfangen eines Elektrokardiogramm-Signals (EKG) und eines Photoplethysmographie-Signals vom Patienten
b. Mittel zur Bestimmung des R-Peaks des EKG-Signals
c. Mittel zur Bestimmung des Fußes des Pulswellensignals
d. Mittel zur Berechnung der Pulsankunftszeit (PAT), d. h. der Zeitspanne zwischen dem R-Peak des EKG-Signals und dem Fuß der Pulswelle des PPG-Signals
e. Mittel zur Berechnung einer geschätzten Pulswellengeschwindigkeit (PWV) durch Kombination des Alters des Patienten, der Karotis-Femoral-Distanz (Dcf) des Patienten und der berechneten Pulsankunftszeit (PAT) in einer Funktion PWVest, um ein Endergebnis zu erhalten,
wobei die Funktion PWVest (Dcf; PAT; Alter) = (Dcf (m) / [PAT (s)) * (k1 * Alter (Jahre) + k2), with
i. $0.05 \leq k 1 \leq 0.15$
ii. $3.5 \leq k 2 \leq 5.0$
iii. wobei das Endergebnis eine geschätzte PWV liefert, ausgedrückt in m/s,
iv. Mittel zum Speichern der geschätzten PWV in einer Datenbank.

10. Vorrichtung nach Anspruch 9, ferner umfassend Mittel zum Schätzen oder Messen des mittleren arteriellen Drucks und Mittel zum Speichern des geschätzten MAPest sowie des Zeitpunkts, zu dem dieser gemessen oder geschätzt wurde.

11. Vorrichtung nach Anspruch 10, die Mittel zum Berechnen der Höhe der dikrotischen Welle (Vpt) zum Zeitpunkt t umfasst, wobei der mittlere arterielle Druck (MAPest) zum Zeitpunkt t durch die Formel MAPest = Calib × Vpt geschätzt wird, wobei Calib = (mittlerer arterieller Druck, gemessen zu einem vorbestimmten Zeitpunkt) / (Höhe der dikrotischen Welle zu dem vorbestimmten Zeitpunkt).

12. Vorrichtung nach einem der Ansprüche 9 bis 11, die ferner Mittel zum Anzeigen oder Exportieren der geschätzten PWV umfasst.

13. Vorrichtung nach einem der Ansprüche 9 bis 12, die ferner Mittel zum Berechnen einer durchschnittlichen PWV durch Berechnung des Mittelwerts der zu verschiedenen Zeitpunkten geschätzten PWV umfasst.

14. Vorrichtung nach Anspruch 13, die ferner Mittel zum Anzeigen oder Exportieren der durchschnittlichen PWV und/oder der niedrigsten geschätzten PWV und/oder der höchsten geschätzten PWV umfasst.

15. Computerprogrammprodukt, das Anweisungen zur Durchführung des Verfahrens gemäß einem der Ansprüche 1 bis 8 umfasst, wenn das Programm auf einem Computer ausgeführt wird und diese Anweisungen von einer logischen Schaltung oder einem Prozessor ausgeführt werden

## Revendications

1. Procédé *ex vivo* mis en œuvre par ordinateur pour évaluer la vitesse de l'onde de pouls (VOP) d'un patient, comprenant la combinaison de l'âge du patient, de la distance carotide-fémorale (Dcf) du patient, exprimée en mètres, et du temps d'arrivée de l'onde de pouls (PAT), exprimé en secondes, dans une fonction PWVest afin d'obtenir un résultat final,
dans lequel la fonction est $PWVest \left(Dcf; PAT; age\right) = \frac{Dcf \left(m\right)}{PAT \left(s\right)} \times \left(k1\times age \left(années\right)+k2\right)$ avec
- $0 , 05 \leq k 1 \leq 0 , 15$
- $3 , 5 \leq k 2 \leq 5,0$
le résultat final fournissant une VOP estimée exprimée en m/s.

2. Procédé *ex vivo* selon la revendication 1, dans lequel la VOP estimée est calculée comme la moyenne d'une pluralité de VOP estimées, calculées sur une période de temps prédéterminée.

3. Procédé ex vivo selon l'une des revendications 1 à 2, dans lequel 0,07 ≤ k1 ≤ 0,086.

4. Procédé ex vivo selon l'une des revendications 1 à 3, dans lequel 4,00 ≤ k2 ≤ 4,25.

5. Procédé ex vivo selon l'une des revendications 1 à 4, dans lequel k1 = 0,079 et k2 = 4,12.

6. Procédé ex vivo selon l'une des revendications 1 à 5, qui est répété à différents instants t afin d'estimer plusieurs valeurs de la vitesse de propagation de l'onde de pouls (PWV) pour le patient.

7. Procédé ex vivo selon l'une des revendications 1 à 6, dans lequel la pression artérielle moyenne du patient est également mesurée ou estimée.

8. Procédé ex vivo selon l'une des revendications 1 à 7, qui est mis en œuvre pendant l'anesthésie du patient.

9. Dispositif destiné à évaluer en continu la vitesse de l'onde de pouls chez un patient, comprenant
a. des moyens pour recevoir un signal d'électrocardiogramme (ECG) et un signal de photopléthysmographie provenant du patient
b. des moyens pour déterminer le pic R du signal ECG
c. des moyens pour déterminer le pied de l'onde de pouls
d. des moyens pour calculer le temps d'arrivée de l'onde de pouls (PAT), qui correspond à la durée entre le pic R du signal ECG et le pied de l'onde de pouls du signal PPG
e. des moyens pour calculer une vitesse estimée de l'onde de pouls (VOP), en combinant l'âge du patient, la distance carotide-fémorale (Dcf) du patient et le temps d'arrivée de l'onde de pouls (PAT) calculé dans une fonction VOPest afin d'obtenir un résultat final,
où la fonction VOPest est définie par (Dcf ; PAT ; âge) = (Dcf (m) / [PAT (s)]) * (k1 * âge (ans) + k2), avec
i. $0 , 05 \leq k 1 \leq 0 , 15$
ii. $3 , 5 \leq k 2 \leq 5,0$
le résultat final fournissant une VOP estimée exprimée en m/s,
f. des moyens pour stocker la VOP estimée dans une base de données.

10. Dispositif selon la revendication 9, comprenant en outre des moyens pour estimer ou mesurer la pression artérielle moyenne (MAP) et des moyens pour stocker la MAPest estimée ainsi que l'instant auquel elle a été mesurée ou estimée.

11. Dispositif selon la revendication 10, qui comprend des moyens pour calculer la hauteur de l'onde dicrote (Vpt) à l'instant t, la pression artérielle moyenne (MAPest) à l'instant t étant estimée par la formule MAPest = Calib × Vpt, où Calib = (pression artérielle moyenne mesurée à un instant prédéterminé) / (hauteur de l'onde dicrote à l'instant prédéterminé).

12. Dispositif selon l'une quelconque des revendications 9 à 11, comprenant en outre des moyens pour afficher ou exporter la VOP estimée.

13. Dispositif selon l'une quelconque des revendications 9 à 12, comprenant en outre des moyens pour calculer une VOP moyenne en calculant la moyenne des VOP estimées à différents instants.

14. Dispositif selon la revendication 13, comprenant en outre des moyens pour afficher ou exporter la VOP moyenne, et/ou la VOP estimée la plus basse et/ou la VOP estimée la plus élevée.

15. Produit logiciel comprenant des instructions pour mettre en œuvre le procédé selon l'une quelconque des revendications 1 à 8, lorsque ledit programme est exécuté sur un ordinateur et que ces instructions sont exécutées par un circuit logique ou un processeur.
